(19) Europäisches Patentamt
European Patent Office
Office européen des brevets

(11) Veröffentlichungsnummer: **0 132 681**
**B1**

(12) # EUROPÄISCHE PATENTSCHRIFT

(45) Veröffentlichungstag der Patentschrift: **03.09.86**

(51) Int. Cl.⁴: **C 07 C 53/15**, C 07 C 51/097

(21) Anmeldenummer: **84108005.4**

(22) Anmeldetag: **09.07.84**

(54) **Verfahren zur Herstellung von Fluorcarbonsäuren.**

(30) Priorität: **21.07.83 DE 3326210**

(43) Veröffentlichungstag der Anmeldung: **13.02.85 Patentblatt 85/7**

(45) Bekanntmachung des Hinweises auf die Patenterteilung: **03.09.86 Patentblatt 86/36**

(84) Benannte Vertragsstaaten: **BE CH DE FR GB IT LI**

(56) Entgegenhaltungen: **Beilsteins Handbuch der Organischen Chemie, 4.Auflage, 2. Ergänzungswerk, 2. Band SPRINGER VERLAG, Berlin, 1942 Seite 186, Zeilen 1-3**

(73) Patentinhaber: **BAYER AG, Konzernverwaltung RP Patentabteilung, D-5090 Leverkusen 1 Bayerwerk (DE)**

(72) Erfinder: **Baasner, Bernd, Dr., Mozartstrasse 41, D-5090 Leverkusen 1 (DE)**
Erfinder: **Klauke, Erich, Dr., Eichendorffweg 8, D-5068 Odenthal (DE)**
Erfinder: **Lantzsch, Reinhard, Dr., Heymannstrasse 32, D-5090 Leverkusen 1 (DE)**

EP 0 132 681 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft die Herstellung von Fluorcarbonsäuren aus Fluornitroaliphaten.

Es ist bekannt, daß man aliphatische Carbonsäuren herstellen kann, wenn man die entsprechenden primären Nitroparaffine mit 85 %iger Schwefelsäure unter Rückfluß kocht (Ind. Eng. Chem. Vol. 31, 118-120 (1939)). Diese bekannte Reaktion läßt sich jedoch nicht auf fluorsubstituierte primären Nitroparaffine übertragen, da unter den dort eingehaltenen Reaktionsbedingungen, insbesondere aufgrund der langen Reaktionszeiten die Fluornitroaliphaten Zersetzung- und Nebenreaktionen eingehen. Besonders treten Oxidationsreaktionen und die Verseifung von in 2-Position gebundenen Halogenatomen auf.

Aus Isvest. Akad. Nauk SSSR, Ser. Khim. 1963, 1798-1799 (engl.) ist bekannt, Trifluoressigsäure herzustellen, indem man eine Mischung von 2, 2, 2-Trifluor-1-nitro-ethan, konzentrierter Schwefelsäure und Wasser unter Schütteln 3 Stunden bei 200° C unter Druck im Autoklaven erhitzt. Dieses Verfahren, das sich nicht auf andere fluorsubstituierte nitroaliphatische Verbindungen übertragen läßt, wird unter sehr drastischen Bedingungen durchgeführt. Dagegen bleibt das 2, 2, 2-Trifluor-1-nitro-ethan unverändert, wenn man die Mischung bei Normaldruck 10 Stunden auf 100° C erhitzt.

Es wurde ein Verfahren zur Herstellung von Fluorcarbonsäuren der Formel

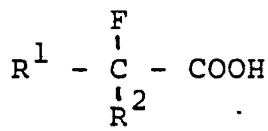

in der

$R^1$ und $R^2$ gleich oder verschieden sind und Wasserstoff, Fluor, Chlor, Brom oder C- bis $C_6$-Alkyl bedeuten, durch Umsetzung von fluornitroaliphatischen Verbindungen der Formel

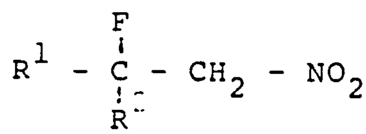

in der $R^1$ und $R^2$ die zuvor angegebene Bedeutung haben, mit Mineralsäuren gefunden, dadurch gekennzeichnet, daß man die Mineralsäure vorlegt und die fluornitroaliphatische Verbindung zudosiert.

Das erfindungsgemäße Verfahren kann durch die folgende Reaktionsgleichung beschrieben werden:

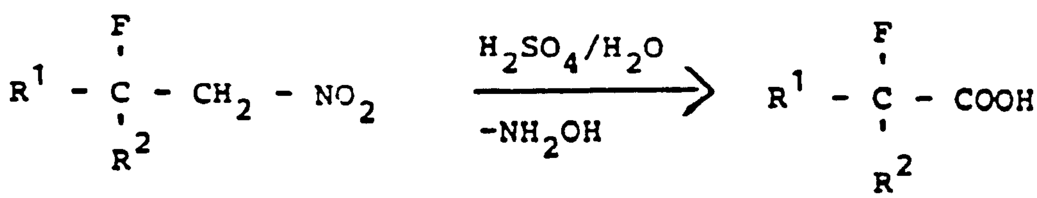


Als Beispiel für $C_1$ bis $C_6$-Alkylreste seien die folgenden Alkylreste genannt: Methyl, Ethyl, Propyl, Isopropyl Butyl, Isobutyl, Pentyl, Isopent Y, Hexyl und Isohexyl. Bevorzugte Alkylreste sind der Methylund der Ethylrest.

Mineralsäuren für das erfindungsgemäße Verfahren sind beispielsweise Schwefelsäure, Chlorwasserstoffsäure, Bromwasserstoffsäure, Phosphorsäure, Salpetersäure und Perchlorsäure.

Bevorzugte Mineralsäuren für das erfindungsgemäße Verfahren sind die Schwefelsäure und Phosphorsäure.

Für die erfindungsgemäße Umsetzung ist Wasser erforderlich, das im allgemeinen zusammen mit der Mineralsäure, als wäßrige Mineralsäure, eingesetzt wird. Der Anteil des Wassers beträgt im allgemeinen 1 bis 10 Mol, bevorzugt 1,2 bis 5 Mol, bezogen auf 1 Mol der fluornitroaliphatischen Verbindung.

Der Anteil der Mineralsäure beträgt im allgemeinen 1 bis 100 Mol, bevorzugt 1 bis 20 Mol, bezogen auf 1 Mol der fluornitroaliphatischen Verbindungen.

Im allgemeinen setzt man die Mineralsäuren mit einem Wassergehalt von 2 bis 20 Gew.-%, bevorzugt von 3 bis 10 Gew.-%, ein.

Für das erfindungsgemäße Verfahren ist ein Überdruck nicht erforderlich.

Das erfindungsgemäße Verfahren wird im Temperaturbereich von 80 bis 250° C, bevorzugt von 100 bis 200° C, durchgeführt.

Fluornitroaliphatische Verbindungen für das erfindungsgemäße Verfahren sind an sich bekannt. Sie können beispielsweise hergestellt werden durch Umsetzung von aliphatischen Nitrogruppen enthaltenden Carbonsäuren mit $CF_4/BF_3$ (Tetrahedron 26, 5737 (1970)) oder durch die Reaktion von Olefinen in wasserfreiem Fluorwasserstoff mit konzentrierter Salpetersäure (Dokt. Akad. Nauk SSSR (149), 222-5 (1963) (engl.) und

Isvest. Akad. Nauk SSSR 1963, 1794-7 (engl.)).

Beispielsweise seien die folgenden fluornitroaliphatischen Verbindungen genannt: 2-Chlor-2, 2-difluornitroethan, 2, 2-Dichlor-2-fluornitroethan, 2, 2-Difluornitro-ethan, 2-Chlor-2-fluornitroethan, 2-Brom-2-fluornitroethan, 2, 2-Difluornitropropan und 2, 2, 2-Trifluornitro-ethan. Bevorzugt für das erfindungsgemäße Verfahren ist 2, 2, 2-Trifluornitroethan.

Das erfindungsgemäße Verfahren kann beispielsweise wie folgt durchgeführt werden:

Die wäßrige Mineralsäure wird vorgelegt und auf die erfindungsgemäße Reaktionstemperatur erwärmt. In diese erwärmte wäßrige Mineralsäure wird die fluornitroaliphatische Verbindung unter Durchmischung eingetropft, eingeleitet, eingepumpt oder auch zusammen mit einem Inertgas wie Stickstoff eingeleitet. Die Dosiergeschwindigkeit bei dieser exotherm verlaufenden Reaktion wird so gewählt, daß die fluoraliphatische Nitroverbindung in dem Maße, wie sie zudosiert wird, auch zu Fluorcarbonsäure abreagiert.

Nach Beendigung der Umsetzung kann die Fluorcarbonsäure bei Normaldruck oder vermindertem Druck (1 bis 0,01 bar) abdestilliert werden. Die Fluorcarbonsäure kann nach Ende der Reaktion auch mittels einer Wasserdampfdestillation oder einer Extraktion mit einem inerten organischen Lösungsmittel, wie Dichlormethan oder Tetrachlormethan isoliert werden.

Der Säuregehalt kann beispielsweise durch Titration bestimmt werden.

Durch Redestillation unter vermindertem Druck oder bei Normaldruck oder durch Umkristallisation kann gegegebenenfalls eine weitere Reinigung erfolgen.

Nach einer bevorzugten Ausführungsform des erfindungsgemäßen Verfahrens wird die bei der Reaktion entstehende Fluorcarbonsäure während der Umsetzung ständig aus dem Reaktionsgemisch, z.B. durch Destillation, entfernt. Thermische Belastungen und somit mögliche Neben- und Zersetzungsreaktionen der Fluorcarbonsäuren können auf diese Weise weitgehend vermieden werden. Durch ständiges, gleichzeitiges Zudosieren von fluornitroaliphatischen Verbindungen und konzentrierter Mineralsäure in einem dafür geeigneten Reaktor kann die Herstellung der Fluorcarbonsäure auch kontinuierlich gestaltet werden.

Aus dem Reaktionsrückstand kann nach den üblichen Verfahren das entstandenen Hydroxylammoniumsulfat isoliert werden. So kann der Rückstand etwa mit Ethanol versetzt werden, so daß das Hydroxylammoniumsulfüt ausfällt. Der Rückstand kann aber auch in Wasser aufgenommen, der Überschuß an Säure neutralisiert und durch Einengen dieses Gemisches das Hydroxylammonium-Sulfat isoliert werden.

Fluorcarbonsäuren können beispielsweise als Zwischenprodukte für Herbizide (DE-OS 2 914 003) verwendet werden. Es ist überraschend, daß nach dem erfindungsgemäßen Verfahren Fluorcarbonsäuren hergestellt werden können, da entgegen den Literaturangaben die Reaktion von 2, 2, 2-Trifluor-1-nitroethan mit Schwefelsäure und Wasser auch bei geringen Temperaturen als 200°C und drucklos durchgeführt werden kann, wodurch die Übertragung dieser Reaktion in einen - auch kontinuierlich durchführbaren technisch - industriellen Maßstab erst möglich wird.

Überraschenderweise erfolgt darüber hinaus nach dem erfindungsgemäßen Verfahren keine Hydrolyse der in 2-Position gebundenen Halogenatome.

**Beispiel 1**

Chlordifluoressigsäure aus 2-Chlor-2, 2-difluornitroethan 220 ml 90 %ige Schwefelsäure wurden auf eine Innentemperatur von 125 - 130°C erhitzt. Unter Rühren wurden in 30 Minuten 72,8 g (0,5 Mol) 2-Chlor-2, 2-difluornitroethan zugetropft, 30 Minuten nachgerührt, dann die Chlordifluoressigsäure bei 114° bis 130°C abdestilliert. Nach Redestillation erhielt man an Produkt bei Kp. 119 bis 122°C 57,6 g (88 %),

$n^{20}_D$: 1.3585.

Nach analoger Verfahrensweise wurden hergestellt:

**Beispiel 2**

Aus 0,5 Mol 2, 2-Dichlor-2-fluornitroethan
60 g (82 %) Dichlorfluoressigsäure, Kp. 156 bis 159°C,
$n^{20}_D$: 1.4171

**Beispiel 3**

Aus 0,5 Mol 2, 2-Difluornitroethan
42,6 g (89 %) Difluoressigsäure, Kp. 133 bis 136°C, $n^{20}_D$: 1.3435.

**Beispiel 4**

Aus 0,5 Mol 2-Chlor-2-fluornitroethan
53,0 g (94 %) Chlorfluoressigsäure, Kp. 74 bis 5° C/25 mbar, $n^{20}_D$: 1.4091.

**Beispiel 5**

Aus 2-Brom-2-fluornitroethan
48,2 g (61 %) Bromfluoressigsäure, Kp. 116 bis 120° C/ 50 mbar, Fp. 49 bis 50° C.

**Beispiel 6**

Aus 2, 2-Difluornitropropan
46,7 g (85 %) Difluorpropionsäure, Kp. 138 bis 140° C, Fp. 39 bis 42° C.

**Beispiel 7**

Trifluoressigsäure aus 2, 2, 2-Trifluornitroethan
180 ml 96 %ige Schwefelsäure wurden vorgelegt und auf 130 bis 135° C Innentemperatur aufgeheizt. Dazu wurden 64,5 g (0,5 Mol) 2, 2, 2-Trifluornitroethan in etwa 150 Minuten zugetropft und gleichzeitig die entstandene Trifluoressigsäure kontinuierlich abdestilliert. Nach Redestillation erhielt man bei Kp. 70 bis 72° C an produkt 46,8 g (82 %).

**Beispiel 8**

Trifluoressigsäure aus 2, 2, 2-Trifluornitroethan
200 ml 95 %ige Schwefelsäure wurden vorgelegt und auf 180-190° C Innentemperatur aufgeheizt. In diese erhitzte Säure wurde durch ein bis zum Boden des Reaktionsgefäßes führendes Einleitungsrohr mit Hilfe eines geringen Stickstoffstromes in etwa 4 Stunden 64,5 g (0,5 Mol) 2, 2, 2-Trifluornitroethan zudosiert. Die entstandene Trifluoressigsäure wurde gleichzeitig kontinuierlich über eine kurze Kolonne abdestilliert. Man erhielt bei Kp. 69,5 - 71° C an Produkt 51,9 g (91 %).

**Patentansprüche**

1. Verfahren zur Herstellung von Fluorcarbonsäuren der Formel

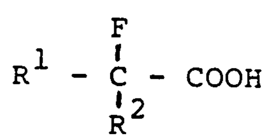

in der
$R^1$ und $R^2$ gleich oder verschieden sind und Wasserstoff, Fluor, Chlor, Brom oder $C_1$- bis $C_6$-Alkyl bedeuten, durch Umsetzung von fluornitroaliphatischen Verbindungen der Formel

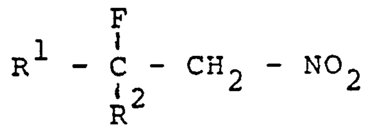

in der $R^1$ und $R^2$ die zuvor angegebene Bedeutung haben, mit Mineralsäuren, dadurch gekennzeichnet, daß man die wäßrige Mineralsäure vorlegt und die fluornitroaliphatische Verbindung zudosiert.

2. Verfahren nach Anspruch 1, dadurch gekennzeichnet, daß es im Temperaturbereich von 80 bis 250°C durchgeführt wird.

3. Verfahren nach den Ansprüchen 1 und 2, dadurch gekennzeichnet, daß die Dosierungsgeschwindigkeit der Geschwindigkeit der Entstehung der Fluorcarbonsaure entspricht.

4. Verfahren nach den Ansprüchen 1 bis 3, dadurch gekennzeichnet, dal man die bei der Umsetzung entstehende Fluorcarbonsäure während der Reaktion ständig aus dem Reaktionigemisch entfernt und gleichzeitig die fluornitroaliphatische Verbindung zudosiert.

## Claims

1. Process for the preparation of fluorocarboxylic acids of the formula

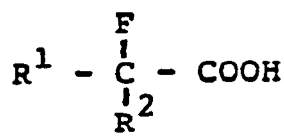

in which

$R^1$ and $R^2$ are identical or different and denote hydrogen, fluorine, chlorine, bromine or $C_1$ to $C_6$ alkyl, by reacting fluoronitroaliphatic compounds of the formula

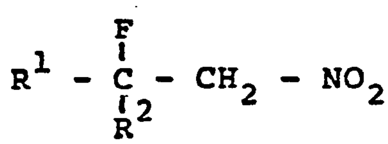

in which

$R^1$ and $R^2$ have the aforementioned meaning, with mineral acids, characterised in that the aqueous mineral acid is initially introduced and the fluoronitroaliphatic compound is metered in.

2. Process according to Claim 1, characterised in that it is carried out in the temperature range of 80 to 250°C.

3. Process according to Claims 1 and 2, characterised in that the rate of metering-in corresponds to the rate of the formation of the fluorocarboxylic acid.

4. Process according to Claims 1 to 3, characterised in that the fluorocarboxylic acid forming in the reaction is

continuously removed from the reaction mixture during the reaction and at the same time the fluoronitroaliphatic compound is metered in.

**Revendications**

1. Procédé de fabrication d'acides fluorocarboxyliques de formule:

$$\begin{array}{ccccc} & & F & & \\ & & | & & \\ R^1 & - & C & - & COOH \\ & & | & & \\ & & R^2 & & \end{array}$$

dans laquelle
$R^1$ et $R^2$ sont identiques ou différents et signifient de l'hydrogène, fluor, chlore, brome ou un alcoyle en $C_1$ à $C_6$,
par réaction de composés fluoronitrés aliphatiques de formule:

$$\begin{array}{ccccccc} & & F & & & & \\ & & | & & & & \\ R^1 & - & C & - & CH_2 & - & NO_2 \\ & & | & & & & \\ & & R^2 & & & & \end{array}$$

dans laquelle $R^1$ et $R^2$ ont la signification indiquée ci-dessus, avec des acides minéraux, caractérisé en ce qu'on introduit d'avance l'acide minéral aqueux et l'on y alimente le composé fluoronitré aliphatique.

2. Procédé selon la revendication 1, caractérisé en ce qu'on l'exécute dans l'intervalle de température de 80 à 250° C.

3. Procédé selon les revendications 1 et 2, caractérisé en ce que la vitesse d'alimentation correspond à la vitesse d'obtention de l'acide fluorocarboxylique.

4. Procédé selon les revendications 1 à 3, caractérisé en ce qu'on élimine constamment pendant la réaction l'acide fluorocarboxylique qui est obtenu dans la réaction à partir du mélange de réaction et en ce que simultanément on alimente le composé fluoronitré aliphatique.